# EUROPEAN PATENT SPECIFICATION

(11) **EP 4 518 811 B1**
(45) Date of publication and mention of the grant of the patent: **24.09.2025**
(21) Application number: 23733616.9
(22) Date of filing: 02.05.2023
(51) Int. Cl.: A61F 2/78, A61F 2/80, A61F 5/01

(54) **ORTHOTIC OR PROSTHETIC DEVICE WITH A SHAPED JOINT**
ORTHESE ODER PROTHESE MIT GEFORMTER VERBINDUNG
DISPOSITIF ORTHÉTIQUE OU PROTHÉTIQUE COMPRENANT UN JOINT FORMÉ

(30) Priority: 06.05.2022 CZ 20220189
(43) Date of publication of application: 12.03.2025
(73) Proprietor: INVENT MEDICAL GROUP, S.R.O., 70800 Ostrava, Pustkovec (CZ)
(72) Inventor: GRYGAR, Ales, 74221 Koprivnice (CZ); ADAMOVSKY, Tomas, 73533 Doubrava (CZ); ROSICKY, Jiri, 73911 Frydlant nad Ostravici, Frydlant (CZ)
(74) Representative: Tomickova, Dana
(86) International application number: PCT/CZ2023/050025
(87) International publication number: WO 2023/213338

(56) References cited:
- WO-A1-98/18414
- CN-A- 112 057 213
- US-A- 1 497 219
- US-A- 2 253 040
- US-A1- 2015 105 867
- US-A1- 2015 351 934
- US-A1- 2018 103 733

## Description

### Field of the Invention

The invention relates to an orthotic or prosthetic device with a shaped joint, in particular prosthetic sockets and limb orthoses, cranial orthoses, trunk orthoses comprising multiple parts made of different materials. For example, prosthetic sockets, upper limb orthoses, lower limb orthoses, trunk orthoses with a peripheral rim made of flexible material, or prosthetic sockets, limb and trunk orthoses with flexible segments inserted into the openings in the shell, or prosthetic sockets with a peripheral rim and an inner liner made of flexible material, or cranial orthoses made of 2 shells - one rigid and one flexible.

### Background of the Invention

Currently, the connection of parts of orthotic or prosthetic devices made of different materials is mainly done by mechanical connection (detachable or non-detachable) or by glued joints.

In prosthetic sockets made of composite material, the connection between the flexible layer of the socket and the rigid layer of the socket is made, for example, by means of a connecting fabric at the boundary between the layers which has good adhesion to the materials of both layers. Such solution is described, for example, in the document WO2021/176165 A1. This is a non-detachable joint, which is technologically demanding, particularly when it comes to the manufacture of tailor-made orthoses or prostheses.

Another known solution for the mechanical joint of two parts of the prosthetic device is described in the document EP3723670 A1, which uses screws connecting the inner soft layer and outer rigid one. This embodiment is structurally complex, wherein the screws considerably complicate the structure of the prosthetic socket as it is necessary to ensure that they interfere as little as possible with the contact with the residual limb.

The document EP2856980 A1 discusses the joint of the inner flexible liner and the outer rigid shell by a layer of silicone adhesive between them. Again, this is a non-detachable joint, which is technologically demanding to manufacture, particularly when it comes to the manufacture of tailor-made orthoses or prostheses.

The document US10271969 B2 discusses the connection of a flexible brim to a rigid socket by the shaped joint, where the prosthetic socket has tapered walls on its top side, wherein this taper complements by its shape the brim, which abuts precisely on the tapered segment of the prosthetic socket from the inner side. This mechanical connection alone does not provide a sufficiently strong mechanical joint, wherein the document suggests that this joint should be additionally secured by gluing or otherwise increasing the adhesion at the point of contact between the socket and the brim.

For example, a purely mechanical joint is presented in the document US20210145613 A1, which describes the connection of the flexible liner and the prosthetic socket by means of projections on the outer side of the liner and openings in the shell of the prosthetic socket. The projections are embodied as backstops which prevent movement of the liner out of the socket by catching on the openings. The liner can be removed from the prosthetic socket by, for example, compressing the liner centrally so that the projections are released from the openings. Such joint is simple to make, is detachable and provides sufficient connection of the parts when the prosthetic socket assembly is worn on the residual limb. However, this solution is not suitable for the connection of prostheses or orthoses with their peripheral rims made of flexible material or with flexible segments inserted into the openings in the shell, that is, the connection of parts of orthotic or prosthetic devices made of different materials which together form one wall of the device. That is to say, this joint only addresses the securing of the liner against being pulled outwards from the prosthetic socket, wherein it is not suitable for connection of two parts which are subjected to the bending stress in the places around the joint.

The document US1497219 A describes a prosthetic socket with a cushioning strip adapted to slidably fit upon the adjacent end of the prosthetic socket. Specifically, the strip is of channel form comprising spaced side walls and a connecting web. This document, however, leaves a room for improvement regarding the stability of the connection between the socket and the cushioning part.

Other prosthetic or orthotic devices with a shape joint are known from documents US20150351934 A1, US20180103733 A1, CN112057213 A, US20150105867 A1, US2253040 A or WO98/18414 A1. For example, the document WO98/18414 A1 discloses a dovetail connection between parts made of materials of different elasticity, however these parts (socket and foot part) together don't form a continuous wall of a prosthetic device.

It therefore follows from the state of the art that it is desirable to develop a solution for the joint of the multi-part orthotic or prosthetic device made of different materials which is simple in structure, detachable and which ensures that the joint is durable both against the pull of the parts away from each other and against mutual bending stresses at the point of the joint.

### Summary of the Invention

The above-mentioned drawbacks are eliminated by an orthotic or prosthetic device with a shaped joint comprising a first part made of a first material and a second part made of a second material connected to the first part, wherein the first material and the second material have different elasticities, where the first part and the second part together form a continuous wall of the orthotic or prosthetic device, and the first part comprises a groove having a mouth in the wall of the first part, wherein the groove bends around a mouth edge of the groove, and the second part comprises a projection following the shape of the groove inserted into the groove.

Such solution for the connection of the first and second parts is simple in structure, allows its detachability and ensures a joint sufficiently durable against both the pull of the parts away from each other and the mutual bending of the parts at the point of the joint.

Preferably, the groove bent between a base of the groove and the mouth of the groove is used. In the preferred embodiment, the groove is bent between the base of the groove and the mouth of the groove by 30 to 90 degrees. By bending the groove along its depth between its base and its mouth, the connection of the parts is made even stronger.

Preferably, the first part is detachably connected to the second part by a projection inserted into the groove. Due to the detachability of the device, it is possible to easily replace the parts and thus easily service the device, and to manipulate the parts more easily, for example for positioning purposes.

Preferably, at least one of the first part and the second part is made by a 3D printing technology. The 3D printing technology enables the manufacture of a part tailored to the respective needs of a given user, wherein the shape of the groove and the corresponding projection can also be adapted to the different shapes of the orthotic or prosthetic device.

In preferred embodiments, the second material is stretchier than the first material. Better results with respect to the durability of the connection and easier detachment/connection of parts occur in embodiments where the projection is made of a stretchier material than the groove. In preferred embodiments, the first material is a construction plastic (PA, PP, PE, CPP, HPP, ABS, PC, PETG, PLA, CF) and the second material is an elastomer (TPU, TPA, TPE, silicone). This is a preferred combination of material properties for a detachable connection of the first and second parts.

Preferably, the groove tapering in the direction from the mouth of the groove to the base of the groove is used. In this way, the groove interferes less with the structure of the first part and additionally, it is easier to insert the projection into the groove.

In preferred embodiments, the first part has an outer wall and an inner wall, wherein the second part at least partially overlaps the first part on both the outer wall and the inner wall. The overlapping portions of the second part provide additional support for the groove-projection connection. More preferably, the first part then includes the groove on its inner wall and the second part includes the corresponding projection on its portion overlapping the inner wall of the first part. With the groove-projection connection on the inner wall of the first part and the overlapping portion of the second part on the outer side, a very durable connection of the parts is achieved while maintaining a simple structure. Such embodiment of the connection of the parts is particularly preferable for attaching the rims to the shell of the device or the stretchy segments in the shell of the device.

In preferred embodiments, the first part has the outer wall and the inner wall, wherein the second part at least partially overlaps the first part on both the outer wall and the inner wall. The overlapping portions of the second part provide additional support for the groove-projection connection. More preferably, the first part then includes the groove on its outer wall and the second part includes the corresponding projection on its portion overlapping the outer wall of the first part. With the groove-projection connection on the outer wall of the first part and the overlapping portion of the second part on the inner side, a very durable connection of the parts is achieved while maintaining a simple structure. Such embodiment of the connection of the parts is particularly preferable for attaching the inner flexible shells (liners) with a circumferential rim to the rigid shell of the device.

Preferably, the first part comprises two elements from a set comprising two grooves and two projections, wherein the second part comprises the other two elements from this set and all the projections are inserted into the corresponding grooves. The pair of groove-projection connections leads to a very strong shaped connection of the parts.

Preferably, the first part includes the groove in its outer wall and the groove in its inner wall and the second part at least partially overlaps both the outer wall and the inner wall of the first part and includes projections inserted into the grooves in both the inner wall and the outer wall of the first part. The groove-projection connections combined with the overlapping of the parts lead to a very durable connection of the parts. Such embodiment of the connection of the parts is particularly preferable for attaching the rims to the shell of the device or the stretchy segments in the shell of the device.

In preferred embodiments, the wall of the first part is narrower on its first side from the mouth of the groove than on its second side from the mouth of the groove, wherein the second part at least partially fills the space above the first side of the wall of the first part by which the first side of the wall of the first part is narrower than the second side of the wall of the first part in such a way that the first part and the second part together form an uniform continuous wall of the orthotic or prosthetic device. When the first and second parts form the uniform continuous wall together, particularly on the inner side with which the user's body is in contact, the user's comfort is enhanced and inadvertent catching of the first or second part at the point of the joint on the external environment is minimised, as the parts do not include protrusions at the point of the joint on which the user's body or various objects could get caught.

Preferably, the first part is the shell of the orthotic or prosthetic device and the second part is the elastic rim lining at least a portion of the circumference of the shell or the stretchy segment inserted into the opening in the shell.

### Description of the Drawings

The summary of the invention is further clarified by the examples of its embodiments, which are described using the attached drawings, in which:

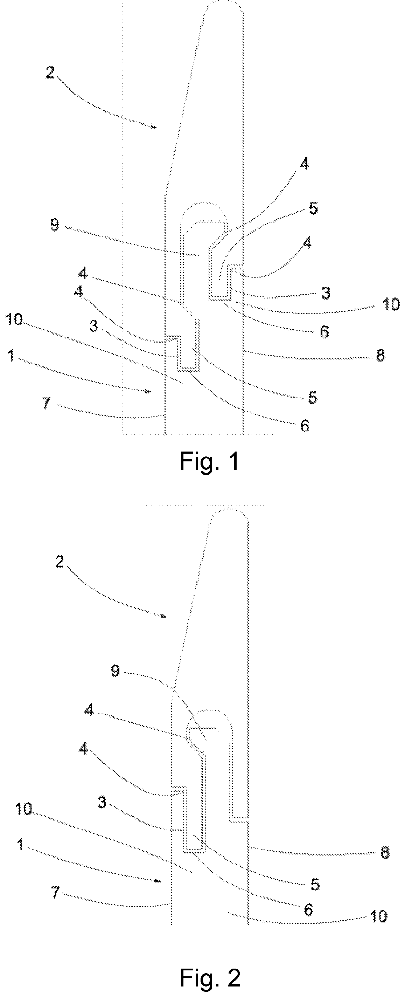

Fig. 1 shows a cross-section of the shaped connection of the parts of the orthotic or prosthetic device according to the first exemplary embodiment of the invention, where the parts are connected via the groove and the projection on both the inner and outer sides of the device,
Fig. 2 shows a cross-section of the shaped connection of the parts of the orthotic or prosthetic device according to the invention, where the parts are connected via the groove and the projection on the inner side of the device, and on the outer side, the second part partially overlaps the first part,

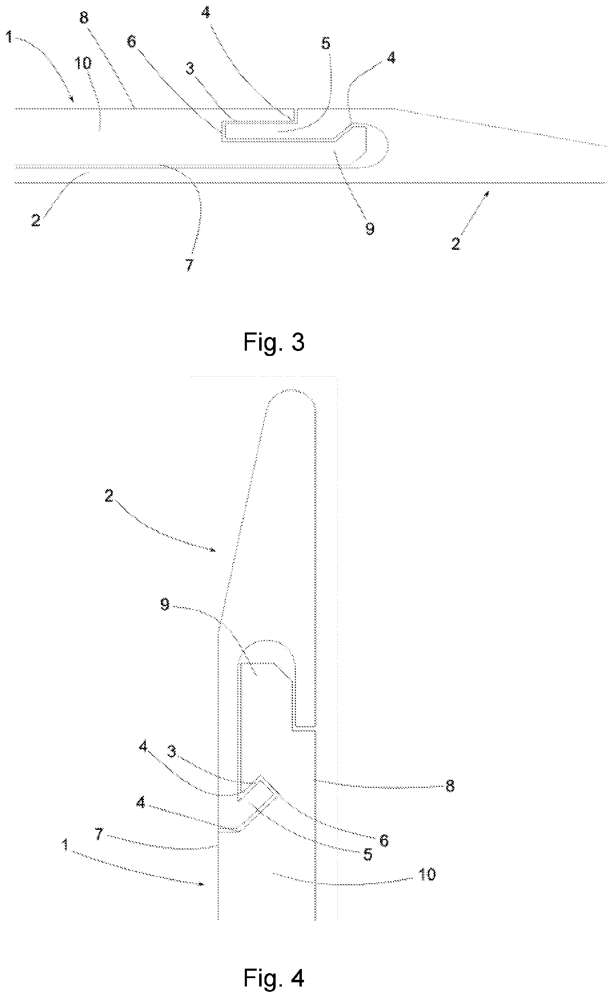

Fig. 3 shows a cross-section of the shaped connection of the parts of the orthotic or prosthetic device according to the invention, where the parts are connected via the groove and the projection on the outer side of the device, and on the inner side, the second part overlaps a larger portion of the first part, this embodiment is, for example, preferable for connecting the inner flexible shell with the outer rigid shell of the cranial orthosis or prosthetic socket,
Fig. 4 shows a cross-section of the shaped connection of the parts of the orthotic or prosthetic device according to the invention, where the parts are connected via the groove and the projection on the inner side of the device, and on the outer side, the second part partially overlaps the first part,

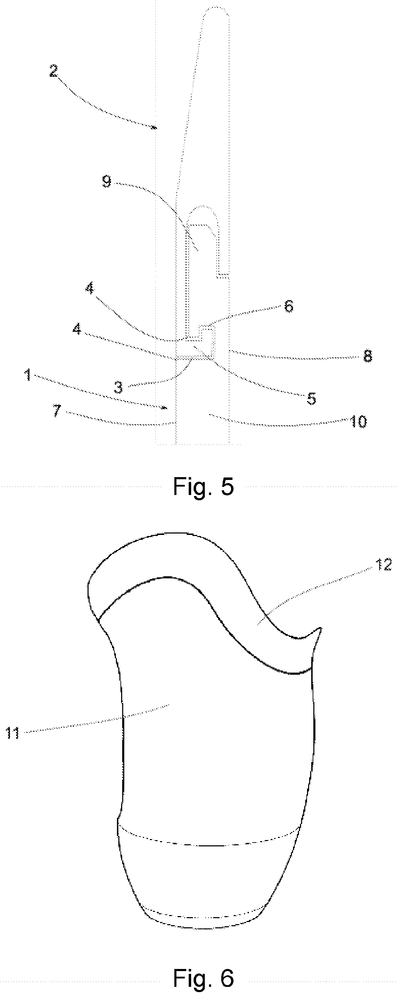

Fig. 5 shows a cross-section of the shaped connection of the parts of the orthotic or prosthetic device according to the invention, where the parts are joined via the groove and the projection on the inner side of the device, and on the outer side, the second part partially overlaps the first part,
Fig. 6 shows the orthotic or prosthetic device with the shaped joint according to the first exemplary embodiment of the invention, where the device is the transtibial prosthetic socket, the first part is the shell of the prosthetic socket, and the second part is the rim of the prosthetic socket, side view,

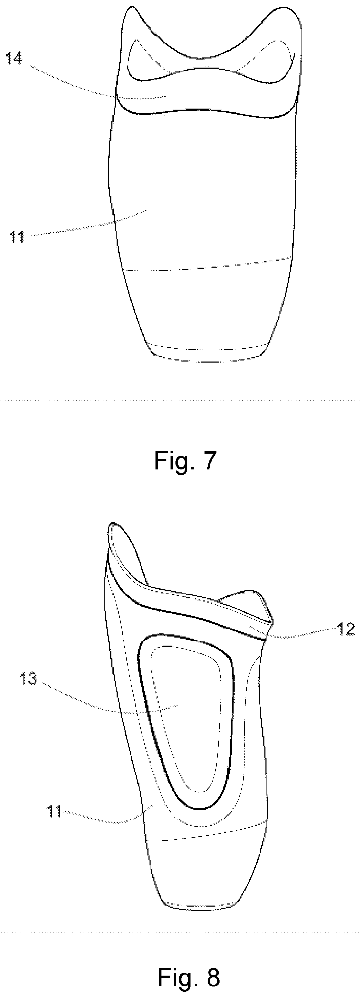

Fig. 7 shows the orthotic or prosthetic device with the shaped joint according to another exemplary embodiment of the invention, where the device is the transtibial prosthetic socket, the first part is the shell of the prosthetic socket, and the second part is the flexible inner liner with the rim of the prosthetic socket, rear view,
Fig. 8 shows the orthotic or prosthetic device with the shaped joint according to the first exemplary embodiment of the invention, where the device is the transfemoral prosthetic socket, the first part is the shell of the socket, and the second part is the rim of the prosthetic socket, and another second part is the stretchy segment in the wall of the socket, front view,

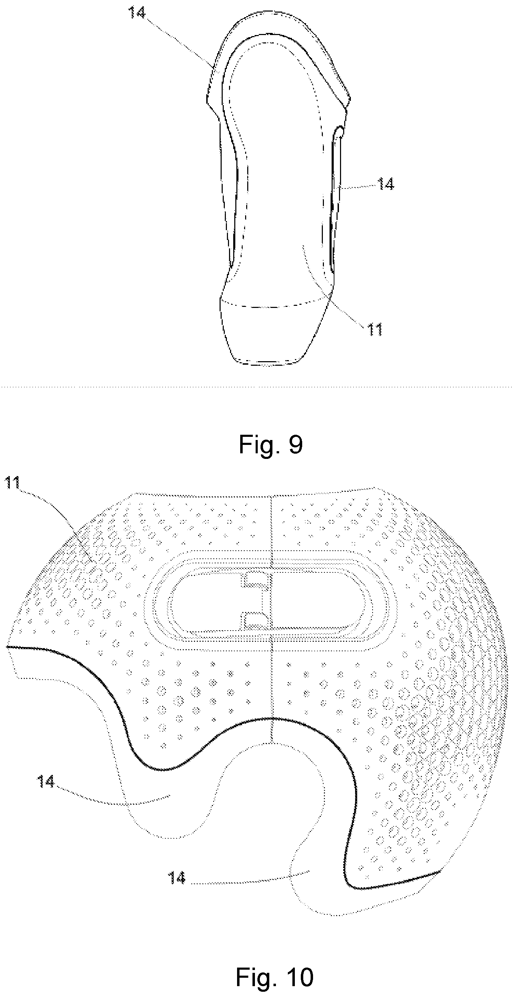

Fig. 9 shows the orthotic or prosthetic device with the shaped joint according to another exemplary embodiment of the invention, where the device is the transfemoral prosthetic socket, the first part is the shell of the socket, and the second part is the flexible inner liner with the rim of the prosthetic socket, side view,
Fig. 10 shows the orthotic or prosthetic device with the shaped joint according to another exemplary embodiment of the invention, where the device is the cranial orthosis, the first part is the shell of the cranial orthosis, and the second part is the inner flexible liner with the rim of the cranial orthosis,

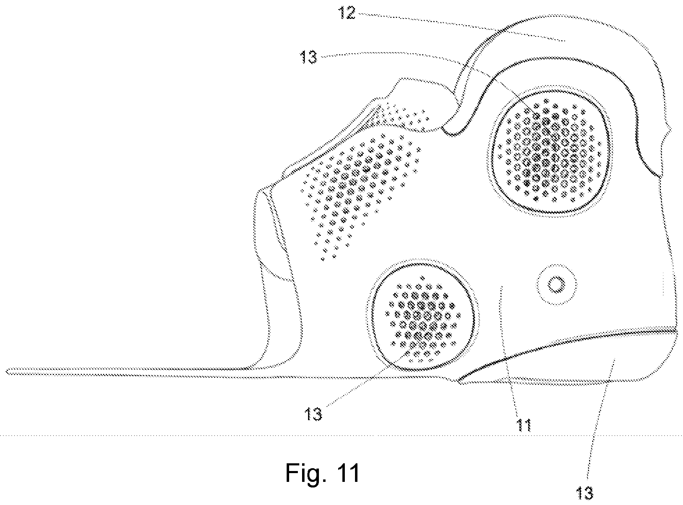

Fig. 11 shows the orthotic or prosthetic device with the shaped joint according to the first exemplary embodiment or different exemplary embodiment of the invention, where the device is the supramalleolar orthosis, the first part is the shell of the supramalleolar orthosis, the second part is the rim of the supramalleolar orthosis, and the other second parts are the stretchy segments inserted into the wall of the orthosis.

### Exemplary Embodiments of the Invention

Some terms will be clarified herein. The groove 3 refers to the longitudinal recess in the wall of the part 1,2. At the surface level of the wall of the part 1,2, the groove 3 is defined by a width and a length, wherein the length of the groove 3 refers to its longest dimension and the width of the groove 3 refers to the dimension of the groove 3 in the direction perpendicular to the longitudinal dimension of the groove 3. The depth of the groove 3 refers to the dimension of the groove 3 perpendicular to the wall area of the part 1,2 or to the mouth of the groove 3. The mouth of the groove 3 refers to the area which the groove 3 occupies in the surface of the wall of the part 1,2 and from which the groove 3 extends into the wall of the part 1,2, wherein the circumference of this area is the mouth edge 4 of the groove. The mouth edge 4 of the groove refers to the border forming the boundary between the surface of the wall of the part 1,2 and the groove 3. By saying that the groove 3 is bent around the mouth edge 4 of the groove, it is meant that at a certain level of its depth the groove 3 extends by its cross-sectional shape beyond the space perpendicularly below the mouth of the groove 3. The straight groove 3 refers to the groove 3 which has straight walls between the base 6 and the mouth of the groove, wherein the opposing walls of the groove 3 may widen the groove 3 in the direction towards the base, taper it, or may be parallel. The cross-section of groove 3 refers to the cross-section in a plane perpendicular to the longitudinal axis of the groove 3. By saying that the projection 5 follows the shape of the groove 3, it is meant that the projection 5 has substantially the same cross-section as the groove 3 along the entire length of the groove 3, wherein after inserting the projection 5 into the groove 3, the projection fills the cavity of the groove 3. By saying that the groove 3 is bent between its base 6 and its mouth, it is meant that the groove 3 is bent in at least one place between its base 6 and its mouth in the direction of its depth, or that the cross-section of the groove 3 has a bent shape, wherein the cross-section of the groove 3 may be, for example, U-, V-, L- or Z-shaped. The outer wall 8 of the first or second part 1,2 refers to the wall area of the first or second part 1,2 oriented away from the user. The inner wall 7 of the first or second part refers to the wall area of the first or second part 1,2 oriented away the user.

An orthotic or prosthetic device may be a transtibial prosthetic socket, transfemoral prosthetic socket, supramalleolar orthosis, ankle joint orthosis, knee orthosis, hip orthosis, wrist orthosis, elbow orthosis, shoulder orthosis, neck orthosis, cranial orthosis, trunk orthosis, etc., but also, for example, a separate portion of an orthosis or prosthesis, such as a flexible inner liner 14 of a prosthetic socket, an anterior flexible part of an ankle joint orthosis, an inner flexible liner 14 of a cranial orthosis.

The orthotic or prosthetic device comprises a first part 1 and a second part 2, which are made of different materials of different elasticities and together form at least a portion of the orthotic or prosthetic device. For example, the first part 1 and the second part 2 may be pairs from the set: a prosthetic socket and its flexible peripheral rim, a prosthetic socket and its inner flexible liner 14 with a rim, an anterior part of an ankle joint orthosis and its top or bottom rim, a shell 11 of an orthosis or prosthesis and a stretchy segment 13 forming a flexible region in the shell 11, a two-part anterior part of a leg orthosis and a flexible joint connecting these parts, a leg orthosis and its elastic rim 12 at the top edge or around the instep, a cranial orthosis and its inner flexible shell with a rim. Thus, the first part 1 and the second part 2 together form a wall of the orthotic or prosthetic device, wherein they may form continuous walls, where the first and second parts 1,2 form a wall in different regions above the surface of the user's body, or they may form layers of the wall, where one part 1,2 forms a more rigid outer shell 11 and the other part 1,2 forms a flexible inner liner 14 providing softer contact of the device with the user.

The first part 1 and the second part 2 are connected by a corresponding groove 3 - projection 5 pair, wherein the first part 1 comprises at least one groove 3 and the second part 2 comprises at least one projection 5. In an embodiment where the first and second parts 1,2 form continuous walls, there are 2 possible locations for such connection. The first variant of the location of the connection of the first and second parts 1,2 is their connection by means of the groove 3 - projection 5 pair on their contact surfaces perpendicular to the surface of the user's body, i.e., at the boundary between the wall of the orthotic or prosthetic device formed by the first part 1 and the wall of the orthotic or prosthetic device formed by the second part 2. The second variant of the location of the connection is possible in cases where the walls of the first part 1 and the second part 2 partially overlap, wherein the second variant of the location of the connection is a connection on the contact surfaces substantially parallel to the surface of the user's body at the point of overlap of the walls of the first and second parts 1,2, in other words, on the adjacent surfaces of the overlapping segments of the first and second parts 1,2. Embodiments comprising the second variant of the location of the connection are shown in Fig. 1 to Fig. 5.

In the second variant of the location of the connection of the first and second parts 1,2, the wall of the first part 1 has a first side 9 and a second side 10 on opposite sides of the groove 3. The first side 9 and the second side 10 of the wall of the first part are adjacent to the groove 3 and form the mouth edge 4 of the groove. The first side 9 of the wall of the first part is to be understood as the side closer to the second part 2, that is, the side closer to the edge of the first part 1 adjacent to the second part 2. The wall of the first part 1 may preferably be narrower on its first side 9 than on its second side 10, wherein the second part 2 partially or fully compensates for the difference in thicknesses of the first and second sides 9,10 of the wall of the first part, wherein it at least partially fills the space by which the wall of the first part 1 is narrower on its first side 9, and the first part 1 and the second part 2 thus form a continuous wall of the orthotic or prosthetic device. In various embodiments, the wall of the first part 1 may be narrower on both sides of the groove 3 than the wall regions a greater distance away from the groove 3, wherein the second part 2 preferably fills the space by which the first part 1 is narrower on both the first and second sides 9,10 of the wall of the first part in such a way that the first part 1 and the second part 2 together form a continuous wall of the orthotic or prosthetic device. The overlapping walls of the first part 1 and the second part 2 may together form a wall of the prosthetic or orthotic device which has a uniform thickness equal to the neighbouring walls where the first and second parts 1,2 do not overlap or where the wall of the prosthetic or orthotic device is formed only by the first or second part 1,2. Such embodiments are shown in Fig. 1 to Fig. 5. In another embodiment, these overlapping walls of the first part 1 and the second part 2 may form a wall with a greater thickness relative to the neighbouring areas where the wall of the prosthetic or orthotic device is formed only by the first or second part 1,2, wherein more space is thereby created for the groove 3 - projection 5 shaped connection , which may thus be stronger.

It is essential for the functionality of the invention that the groove 3 is bent around the mouth edge 4 of the groove. This can be achieved, for example, by the groove 3 being straight between its base 6 and its mouth, wherein from the mouth of the groove 3 the groove 3 extends into the wall of the first part 1 at an angle of at least 5° relative to the mouth of the groove 3, preferably an angle of 15° to 70°, more preferably 30° to 50°. In this embodiment, the groove 3 is preferably, in the second variant of the location of the connection, bent around the mouth edge 4 of the groove, which is formed by the first side 9 of the wall of the first part, wherein the first side 9 of the wall of the first part and the groove 3 together form a sharp border, behind which the projection 5 is wedged which follows the shape of the groove 3. The groove 3 according to this embodiment is shown in Fig. 4. When the first and second parts 1,2 pull away from each other or the first and second parts 1,2 bend relative to each other, the second part 2 catches on this border formed by the first side 9 of the wall of the first part and the groove 3, wherein the projection 5 is wedged in the groove 3, thereby maintaining the connection of the first and second parts 1,2. This embodiment of the groove 3 and the projection 5 is particularly preferable in the case of the second variant of the location of the connection, wherein such embodiment is shown in Fig. 4.

Another possible embodiment of the groove 3 bent around the mouth edge 4 of the groove is one where the groove 3 is bent around its mouth or is bent between its base 6 and its mouth. Thus, in its portion adjacent to the mouth, the groove 3 of such embodiment may be oriented perpendicularly to the mouth or to the wall of the first part 1, wherein deeper in the wall of the first part 1 below its mouth, the groove 3 is bent by at least 5°, preferably by 15° to 120°, more preferably by 30° to 90°, with respect to this portion of itself. It can also be said that the groove 3 is bent relative to an axis perpendicular to the mouth of the groove 3. This bend below the level of the mouth of the groove 3 ensures that the groove 3 bends beyond the mouth edge 4 of the groove, or below the groove mouth edge 4 when looking perpendicular to the mouth of the groove 3. Thus, the groove 3 may be V-, U-, or L-shaped, wherein the base 6 of the groove is offset relative to the mouth when looking perpendicular to the wall of the first part 1. The corresponding projection 5 following the shape of such groove 3 wedges in the groove 3 when the first part 1 and the second part 2 pull away from each other or the first and second parts 1,2 bend relative to each other, thereby maintaining the connection of the first and second parts 1,2. This is because the included shapes of the groove 3 and the projection 5, in combination with their close contact, prevent the projection 5 from falling out of the groove 3, since for example in the case of the second part 2 being made of a more elastic material, it is necessary to exceed the force required to bend the projection 5 through the bent portion of the groove 3. These embodiments of the grooves 3 are shown in Figs. 1, 2, 3 and 5. In other embodiments, the groove 3 may be bent along its depth between its base 6 and its mouth 2 or more times, wherein it may be Z-shaped, for example, or even have a shape of the zig-zag structure at higher numbers of bends of the groove 3.

Further embodiments of the groove 3 and the projection 5 according to the invention are combinations of the two embodiments described in the previous two paragraphs. In such embodiment, the groove 3 thus extends into the wall of the first part 1 at a certain angle with respect to its mouth, while at the same time being bent in at least one place between its mouth and its base 6.

For the strongest possible connection durable against the pull of the first and second parts 1,2 away from each other, it is preferable if the groove 3 is bent towards the first side 9 of the wall of the first part in the case of the second variant of the location of the connection, so that the base 6 of the groove is located underneath the first side 9 of the wall of the first part (Fig. 4 and Fig. 5). The shape of the groove 3, and therefore of the projection 5, can be likened in such embodiment to a hook which holds the parts together by being hooked on the first side 9 of the wall of the first part. On the other hand, for the durability of the joint against forces bending the first and second parts 1,2 relative to each other, it is preferable if the groove 3 is bent towards the second side 10 of the wall of the first part, so that the base 6 of the groove is underneath the second side 10 of the wall of the first part (embodiment in Figs. 1, 2 and 3). During bending, the projection 5 is wedged against the second side 10 of the wall of the first part or the wall of the groove 3 on the opposite side of the groove to the second side 10 of the wall of the first part, thereby holding the parts 1,2 together and preventing the projection 5 from being pulled out of the groove 3.

In order to provide a stronger connection, the first part 1 and the second part 2 may be joined by more than one groove 3 - projection 5 connection, wherein the first part 1 may, for example, comprise two grooves 3 or one groove 3 and one projection 5 and the second part 2 may comprise two projections 5 or one projection 5 and one groove 3. In such embodiments, the first and second variation of the location of the groove 3 - projection 5 connection may be used in combination. Thus, for example, the first part 1 may comprise one groove 3 on the inner wall 7 and one groove 3 on the outer wall 8, wherein the second part 2 overlaps both the inner and outer walls 7,8 of the first part and it comprises projections 5 oriented towards the corresponding grooves 3 on the both overlapping segments, the parts 1,2 are thus connected by a pair of connections according to the first location variant, such embodiment is shown in Fig. 1. In other embodiments, the first location variant may be used in combination with the second location variant. For example, in some embodiments, the first part 1 may comprise one groove 3 on the wall oriented perpendicular to the surface of the patient's body and the second groove 3 on its inner wall 7 parallel to the surface of the patient's body, wherein the second part 2 comprises one corresponding projection 5 on the wall oriented perpendicular to the surface of the patient's body adjacent to the wall of the first part 1 with the groove 3 and the second corresponding projection 5 oriented toward the inner wall 7 of the first part on the overlapping segment. Other embodiments may be similarly constructed, the only difference being that the first part 1 comprises one groove 3 and one projection 5 and the second part 2 comprises the second groove 3 and the second projection 5.

In other embodiments of the invention, the mouth of the groove 3 may represent a taper, wherein the groove 3 widens beyond the mouth, thereby bending beyond the mouth edge 4 of the groove. The groove 3 may widen up to its base 6, where the base represents the widest portion of the groove 3 along its depth, or it may widen up to its widest portion along its depth and then taper again towards its base 6, the cross-section of the groove 3 may thus, for example, have a rounded shape. In these embodiments, the tapered mouth of the groove 3 represents a barrier preventing the projection 5 inserted into the groove 3 from sliding out, as it is narrower than the projection 5 inserted into the groove 3 beyond the mouth.

The first part 1 is made of a first material and the second part 2 is made of a second material. The first and second materials differ in their elasticity, and thus one of these materials more easily undergoes reversible deformation under external forces. The first and second materials are preferably suitable for the 3D printing technology, for example SLA, SLS, FDM, MJF, DLP, 3DP, PJF, CLIP. The material may be PA, ABS, PLA, PE, PP, CPP, HPP, PC, PETG, photopolymers, elastomers (TPU, TPA, TPE, silicones) and other materials suitable not only for the 3D printing methods mentioned above. Other methods of manufacturing the parts 1,2 may also be considered, for example, casting or injection moulding, wherein the material may be also a composite strengthened with glass fibres, carbon fibres, carbon nanofibers, or any other suitable fibres.

In some embodiments, the first material may exhibit higher elasticity than the second material, wherein the first part 1 with at least one groove 3 is more flexible than the second part 2 with at least one projection 3. Alternatively, in other embodiments, the second material may exhibit higher elasticity. It is irrelevant to the functionality of the invention whether the projection 5 or the groove 5 is made of the more elastic material, what is important is that at least one element of the set comprising the groove 3 and the projection 5 is made of more elastic material with respect to the other. If the projection 5 is more elastic, its insertion into the groove 3 is facilitated by its elasticity, as its shape adapts to the bending of the groove 3; conversely, if the groove 3 (or the first part 1) is more elastic, its opening is facilitated, wherein after the insertion of the stiffer projection 5 the groove 3 contracts again, thereby securing the projection 5.

The elasticity of the first or second material is determined in the context of the invention by the tensile modulus of elasticity measured according to the ASTM D638 standard of December 2014. Preferably, the difference in the tensile modulus of elasticity between the first and second material is at least 400 MPa, more preferably 800 MPa. In preferred embodiments, the tensile modulus of elasticity of the stiffer material is 1,000 to 4,000 MPa at room temperature and the tensile modulus of elasticity of the more elastic material is 3 to 200 MPa at room temperature. By saying that the first material and the second material have different elasticities, it is meant that they have different tensile modulus of elasticity. In embodiments where one of the first and second materials is an elastomer, the tensile modulus of elasticity thereof is preferably measured according to the DIN53504 standard of March 2017.

Specific examples of embodiments of the orthotic or prosthetic device comprising the shaped connection according to the invention with reference to the corresponding drawings. Exemplary embodiments are shown in Figs. 6 to 11.

The first exemplary embodiment is the transtibial prosthetic socket shown in Fig. 6 with the shaped connection, where the first and second parts 1,2 are connected by the groove 3 and the projection 5 on the inner and outer sides 7,8 of the device, shown in Fig. 1. In the first exemplary embodiment, the first part 1 is the shell 11 of the prosthetic socket and the second part 2 is the elastic rim 12 of the prosthetic socket. The second material has a higher elasticity than the first material, wherein the first material is a polyamide, and the second material is a thermoplastic polyurethane. The groove 3 in the inner wall 7 and the groove 3 in the outer wall 8 of the first part according to Fig. 1 are located around the entire top peripheral circumference of the shell 11 of the prosthetic socket, wherein the elastic circumferential rim 12 comprises respective projections 5 inserted into the grooves 3 around their entire circumference.

The second exemplary embodiment is the transtibial prosthetic socket shown in Fig. 7 with the shaped connection, where the first and second parts 1,2 are connected by the groove 3 and the projection 5 only on the outer side 8 of the device according to the embodiment in Fig. 3. The first part 1 is the shell 11 of the prosthetic socket and the second part 2 is the elastic inner liner 14 with the rim 12 of the prosthetic socket. The second material has a higher elasticity than the first material, wherein the first material is a polyamide, and the second material is a thermoplastic polyurethane. The groove 3 in the outer wall 8 of the first part according to Fig. 3 is located around the entire top peripheral circumference of the shell 11 of the prosthetic socket, wherein the elastic circumferential rim 12 comprises the corresponding projection 5 inserted into the groove 3 around its entire circumference.

In the third exemplary embodiment, the device is the transfemoral prosthetic socket, the first part 1 is the shell 11 of the socket, the first material is a polyamide, and the second material is a thermoplastic polyurethane. The device according to the third exemplary embodiment comprises a total of 2 second parts 2, where one second part represents the rim 12 of the prosthetic socket and another second part 2 represents the stretchy segment 13 in the wall of the shell 11. This embodiment is shown in Fig. 8. The elastic circumferential rim 12 is connected to the shell 11 of the prosthetic socket by the shaped connection according to the invention shown in Fig. 1 around its entire circumference, and the stretchy segment 13 in the wall of the shell 11 is connected to the shell 11 by the shaped connection according to the invention shown in Fig. 1 or Fig. 2 around its entire edge, by which it connects to the wall of the shell 11.

In the fourth exemplary embodiment, the device is the transfemoral prosthetic socket, the first part 1 is the shell 11 of the socket, the first material is a polyamide, and the second material is a thermoplastic polyurethane. The device according to the second exemplary embodiment comprises the second part 2, which is the inner flexible liner 14 that also forms the rim 12 of the prosthetic socket. This embodiment is shown in Fig. 9. The elastic circumferential rim 12 is connected to the shell 11 of the prosthetic socket by the shaped connection according to the invention shown in Fig. 3 around its entire edge, by which it connects to the wall of the shell 11.

In the fifth exemplary embodiment, the orthotic or prosthetic device is the cranial orthosis, the first part 1 is the shell 11 of the cranial orthosis and the second part *2* is the inner flexible liner 14 forming the rim 12 of the cranial orthosis, wherein the inner flexible liner 14 is connected to the shell 11 by the shaped connection according to the invention shown in Fig. 3.

In the sixth exemplary embodiment shown in Fig. 11, the orthotic or prosthetic device is the supramalleolar orthosis and the first part 1 is the shell 11 of the supramalleolar orthosis. The device according to the sixth exemplary embodiment comprises a total of 3 second parts 2, where one second part 2 represents the top peripheral rim 12 of the supramalleolar orthosis and the other 2 second parts 2 represent 2 separate stretchy segments 13 in the wall of the supramalleolar orthosis.

### Industrial Applicability

The invention may also be used in orthotic and prosthetic devices as a fastening mechanism, where the shaped connection is detached for the purpose of putting on or attaching the device and connected for the purpose of locking the device in place on the user's body.

### List of Reference Numerals

1 - First part
2 - Second part
3 - Groove
4 - Mouth edge of the groove
5 - Projection
6 - Base of the groove
7 - Inner wall
8 - Outer wall
9 - First side of the wall of the first part
10 - Second side of the wall of the first part
11 - Shell
12 - Elastic rim
13 - Stretchy segment
14 - Inner flexible liner with a rim

## Claims

1. An orthotic or prosthetic device with a shaped joint comprising a first part (1) made of a first material and a second part (2) made of a second material connected to the first part (1), wherein the first material and the second material have different elasticities, **characterized in that** the first part (1) and the second part (2) together form a continuous wall of the orthotic or prosthetic device, wherein the first part (1) comprises a groove (3) having a mouth in the wall of the first part (1), wherein the groove (3) is bent around a mouth edge (4) of the groove, and the second part (2) comprises a projection (5) following the shape of the groove (3) inserted into the groove (3).

2. The orthotic or prosthetic device with the shaped joint according to claim 1, **characterized in that** the groove (3) is bent between a base (6) of the groove and the mouth of the groove (3).

3. The orthotic or prosthetic device with the shaped joint according to claim 2, **characterized in that** the groove (3) is bent by 30 to 90 degrees between the base (6) of the groove and the mouth of the groove (3).

4. The orthotic or prosthetic device with the shaped joint according to any of the preceding claims, **characterized in that** the first part (1) is detachably connected to the second part by the projection (5) inserted into the groove (3).

5. The orthotic or prosthetic device with the shaped joint according to any of the preceding claims, **characterized in that** at least one of the first part (1) and the second part (2) is manufactured by a 3D printing technology.

6. The orthotic or prosthetic device with the shaped joint according to any of the preceding claims, **characterized in that** the second material is more elastic than the first material.

7. The orthotic or prosthetic device with the shaped joint according to claim 6, **characterized in that** the first material is a construction plastic and the second material is an elastomer.

8. The orthotic or prosthetic device with the shaped joint according to any of the preceding claims, **characterized in that** the groove (3) tapers in the direction from the mouth of the groove (3) to the base (6) of the groove.

9. The orthotic or prosthetic device with the shaped joint according to any of the preceding claims, **characterized in that** the first part (1) has an outer wall (8) and an inner wall (7), wherein the second part (2) at least partially overlaps the first part (1) on both the outer wall (8) and the inner wall (7).

10. The orthotic or prosthetic device with the shaped joint according to claim 9, **characterized in that** the first part (1) comprises the groove (3) on its outer wall (8) and the second part (2) comprises the corresponding projection (5) on its portion overlapping the outer wall (8) of the first part.

11. The orthotic or prosthetic device with the shaped joint according to any of the preceding claims, **characterized in that** the first part (1) comprises two elements from a set comprising two grooves (3) and two projections (5), wherein the second part (2) comprises the other two elements from this set and all the projections (5) are inserted into the corresponding grooves (3).

12. The orthotic or prosthetic device with the shaped joint according to claim 11, **characterized in that** the first part (1) comprises the groove (3) in its inner wall (7) and the groove (3) in its outer wall (8) and the second part (2) at least partially overlaps both the inner wall (7) and the outer wall (8) of the first part and comprises projections (5) inserted into the grooves (3) on both the inner wall (7) and the outer wall (8) of the first part.

13. The orthotic or prosthetic device with the shaped joint according to any of the preceding claims, **characterized in that** the wall of the first part (1) is narrower on its first side (9) from the mouth of the groove (3) than on its second side (10) from the mouth of the groove (3), wherein the second part (2) at least partially fills the space above the first side (9) of the wall of the first part by which the first side (9) of the wall of the first part is narrower than the second side (10) of the wall of the first part, so that the first part (1) and the second part (2) together form a uniform continuous wall of the orthotic or prosthetic device.

14. The orthotic or prosthetic device with the shaped joint according to any of the preceding claims, **characterized in that** the first part (1) is a shell (11) of the orthotic or prosthetic device and the second part (2) is an elastic rim (12) lining at least a portion of the circumference of the shell (11) or a stretchy segment (13) inserted into an opening in the shell (11).

15. The orthotic or prosthetic device with the shaped joint according to any of the preceding claims, **characterized in that** the first part (1) is the shell (11) of the orthotic or prosthetic device and the second part (2) is an internal flexible liner (14) with the rim lining at least a portion of the circumference of the shell (11).

## Patentansprüche

1. Eine orthetische oder prothetische Vorrichtung mit einem geformten Gelenk, umfassend einen ersten Teil (1) aus einem ersten Material und einen mit dem ersten Teil (1) verbundenen zweiten Teil (2) aus einem zweiten Material, wobei das erste Material und das zweite Material unterschiedliche Elastizitäten aufweisen, **dadurch gekennzeichnet, dass** der erste Teil (1) und der zweite Teil (2) zusammen eine durchgehende Wand der orthetischen oder prothetischen Vorrichtung bilden, wobei der erste Teil (1) eine Nut (3) mit einer Mündung in der Wand des ersten Teils (1) umfasst, wobei die Nut (3) um einen Mündungsrand (4) der Nut gebogen ist und der zweite Teil (2) einen Vorsprung (5) umfasst, der der Form der Nut (3) folgt und in die Nut (3) eingesetzt ist.

2. Die orthetische oder prothetische Vorrichtung mit dem geformten Gelenk nach Anspruch 1, **dadurch gekennzeichnet, dass** die Nut (3) zwischen einer Basis (6) der Nut und der Mündung der Nut (3) gebogen ist.

3. Die orthetische oder prothetische Vorrichtung mit dem geformten Gelenk nach Anspruch 2, **dadurch gekennzeichnet, dass** die Nut (3) zwischen der Basis (6) der Nut und der Mündung der Nut (3) um 30 bis 90 Grad gebogen ist.

4. Die orthetische oder prothetische Vorrichtung mit dem geformten Gelenk nach einem der vorstehenden Ansprüche, **dadurch gekennzeichnet, dass** der erste Teil (1) durch den in die Nut (3) eingesetzten Vorsprung (5) lösbar mit dem zweiten Teil verbunden ist.

5. Die orthetische oder prothetische Vorrichtung mit dem geformten Gelenk nach einem der vorstehenden Ansprüche, **dadurch gekennzeichnet, dass** mindestens eines der ersten Teile (1) und der zweiten Teile (2) mittels einer 3D-Drucktechnologie hergestellt ist.

6. Die orthetische oder prothetische Vorrichtung mit dem geformten Gelenk nach einem der vorstehenden Ansprüche, **dadurch gekennzeichnet, dass** das zweite Material elastischer ist als das erste Material.

7. Die orthetische oder prothetische Vorrichtung mit dem geformten Gelenk nach Anspruch 6, **dadurch gekennzeichnet, dass** das erste Material ein Konstruktionskunststoff und das zweite Material ein Elastomer ist.

8. Die orthetische oder prothetische Vorrichtung mit dem geformten Gelenk nach einem der vorstehenden Ansprüche, **dadurch gekennzeichnet, dass** sich die Nut (3) in Richtung von der Mündung der Nut (3) zur Basis (6) der Nut verjüngt.

9. Die orthetische oder prothetische Vorrichtung mit dem geformten Gelenk nach einem der vorstehenden Ansprüche, **dadurch gekennzeichnet, dass** der erste Teil (1) eine Außenwand (8) und eine Innenwand (7) aufweist, wobei der zweite Teil (2) den ersten Teil (1) sowohl an der Außenwand (8) als auch an der Innenwand (7) zumindest teilweise überlappt.

10. Die orthetische oder prothetische Vorrichtung mit dem geformten Gelenk nach Anspruch 9, **dadurch gekennzeichnet, dass** der erste Teil (1) die Nut (3) an seiner Außenwand (8) umfasst und der zweite Teil (2) den entsprechenden Vorsprung (5) an seinem Abschnitt, der die Außenwand (8) des ersten Teils überlappt, umfasst.

11. Die orthetische oder prothetische Vorrichtung mit dem geformten Gelenk nach einem der vorstehenden Ansprüche, **dadurch gekennzeichnet, dass** der erste Teil (1) zwei Elemente aus einer Gruppe umfassend zwei Nuten (3) und zwei Vorsprünge (5) umfasst, wobei der zweite Teil (2) die beiden anderen Elemente aus dieser Gruppe umfasst und alle Vorsprünge (5) in die entsprechenden Nuten (3) eingesetzt sind.

12. Die orthetische oder prothetische Vorrichtung mit dem geformten Gelenk nach Anspruch 11, **dadurch gekennzeichnet, dass** der erste Teil (1) die Nut (3) in seiner Innenwand (7) und die Nut (3) in seiner Außenwand (8) umfasst und der zweite Teil (2) sowohl die Innenwand (7) als auch die Außenwand (8) des ersten Teils zumindest teilweise überlappt und Vorsprünge (5) umfasst, die in die Nuten (3) sowohl an der Innenwand (7) als auch an der Außenwand (8) des ersten Teils eingesetzt sind.

13. Die orthetische oder prothetische Vorrichtung mit dem geformten Gelenk nach einem der vorstehenden Ansprüche, **dadurch gekennzeichnet, dass** die Wand des ersten Teils (1) an ihrer ersten Seite (9) von der Mündung der Nut (3) schmaler als an ihrer zweiten Seite (10) von der Mündung der Nut (3) ist, wobei der zweite Teil (2) den Raum über der ersten Seite (9) der Wand des ersten Teils zumindest teilweise ausfüllt, um den die erste Seite (9) der Wand des ersten Teils schmaler als die zweite Seite (10) der Wand des ersten Teils ist, so dass der erste Teil (1) und der zweite Teil (2) zusammen eine gleichmäßige durchgehende Wand der orthetischen oder prothetischen Vorrichtung bilden.

14. Die orthetische oder prothetische Vorrichtung mit dem geformten Gelenk nach einem der vorstehenden Ansprüche, **dadurch gekennzeichnet, dass** der erste Teil (1) eine Schale (11) der orthetischen oder prothetischen Vorrichtung ist und der zweite Teil (2) eine elastische Kante (12) ist, die mindestens einen Abschnitt des Umfangs der Schale (11) auskleidet, oder ein dehnbares Segment (13), das in eine Öffnung in der Schale (11) eingesetzt ist.

15. Die orthetische oder prothetische Vorrichtung mit dem geformten Gelenk nach einem der vorstehenden Ansprüche , **dadurch gekennzeichnet, dass** der erste Teil (1) die Schale (11) der orthetischen oder prothetischen Vorrichtung ist und der zweite Teil (2) eine innere flexible Auskleidung (14) ist, deren Kante zumindest einen Abschnitt des Umfangs der Schale (11) auskleidet.

## Revendications

1. Un dispositif orthétique ou prothétique avec un joint formé, comprenant une première partie (1) en un premier matériau et une seconde partie (2) en un second matériau relié à la première partie (1), où le premier matériau et le second matériau ont des élasticités différentes, **caractérisé en ce que** la première partie (1) et la seconde partie (2) forment ensemble une paroi continue du dispositif orthétique ou prothétique, où la première partie (1) comprend une rainure (3) avec une embouchure dans la paroi de la première partie (1), où la rainure (3) est pliée autour d'un bord d'embouchure (4) de la rainure, et la seconde partie (2) comprend une saillie (5) suivant la forme de la rainure (3) insérée dans la rainure (3).

2. Le dispositif orthétique ou prothétique avec un joint formé selon la revendication 1, **caractérisé en ce que** la rainure (3) est pliée entre une base (6) de la rainure et l'embouchure de la rainure (3).

3. Le dispositif orthétique ou prothétique avec un joint formé selon la revendication 2, **caractérisé en ce que** la rainure (3) est pliée de 30 à 90 degrés entre la base (6) de la rainure et l'embouchure de la rainure (3).

4. Le dispositif orthétique ou prothétique avec un joint formé selon l'une quelconque des revendications précédentes, **caractérisé en ce que** la première partie (1) est reliée de manière amovible à la seconde partie par la saillie (5) insérée dans la rainure (3).

5. Le dispositif orthétique ou prothétique avec un joint formé selon l'une quelconque des revendications précédentes, **caractérisé en ce qu'**au moins l'une de la première partie (1) et de la seconde partie (2) est fabriquée par une technologie d'impression 3D.

6. Le dispositif orthétique ou prothétique avec un joint formé selon l'une quelconque des revendications précédentes, **caractérisé en ce que** le second matériau est plus élastique que le premier matériau.

7. Le dispositif orthétique ou prothétique avec un joint formé selon la revendication 6, **caractérisé en ce que** le premier matériau est un plastique de construction et le second matériau est un élastomère.

8. Le dispositif orthétique ou prothétique avec un joint formé selon l'une quelconque des revendications précédentes, **caractérisé en ce que** la rainure (3) se rétrécit dans la direction allant de l'embouchure de la rainure (3) à la base (6) de la rainure.

9. Le dispositif orthétique ou prothétique avec un joint formé selon l'une quelconque des revendications précédentes, **caractérisé en ce que** la première partie (1) a une paroi extérieure (8) et une paroi intérieure (7), où la seconde partie (2) chevauche au moins partiellement la première partie (1) à la fois sur la paroi extérieure (8) et sur la paroi intérieure (7).

10. Le dispositif orthétique ou prothétique avec un joint formé selon la revendication 9, **caractérisé en ce que** la première partie (1) comprend la rainure (3) sur sa paroi extérieure (8) et la seconde partie (2) comprend la saillie correspondante (5) sur sa portion chevauchant la paroi extérieure (8) de la première partie.

11. Le dispositif orthétique ou prothétique avec un joint formé selon l'une quelconque des revendications précédentes, **caractérisé en ce que** la première partie (1) comprend deux éléments d'un ensemble comprenant deux rainures (3) et deux saillies (5), où la seconde partie (2) comprend les deux autres éléments de cet ensemble et toutes les saillies (5) sont insérées dans les rainures (3) correspondantes.

12. Le dispositif orthétique ou prothétique avec un joint formé selon la revendication 11, **caractérisé en ce que** la première partie (1) comprend la rainure (3) dans sa paroi intérieure (7) et la rainure (3) dans sa paroi extérieure (8) et la seconde partie (2) chevauche au moins partiellement la paroi intérieure (7) et la paroi extérieure (8) de la première partie et comprend des saillies (5) insérées dans les rainures (3) à la fois sur la paroi intérieure (7) et sur la paroi extérieure (8) de la première partie.

13. Le dispositif orthétique ou prothétique avec un joint formé selon l'une quelconque des revendications précédentes, **caractérisé en ce que** la paroi de la première partie (1) est plus étroite sur son premier côté (9) à partir de l'embouchure de la rainure (3) que sur son second côté (10) à partir de l'embouchure de la rainure (3), où la seconde partie (2) remplit au moins partiellement l'espace au-dessus du premier côté (9) de la paroi de la première partie par lequel le premier côté (9) de la paroi de la première partie est plus étroit que le second côté (10) de la paroi de la première partie, de sorte que la première partie (1) et la seconde partie (2) forment ensemble une paroi continue uniforme du dispositif orthétique ou prothétique.

14. Le dispositif orthétique ou prothétique avec un joint formé selon l'une quelconque des revendications précédentes, **caractérisé en ce que** la première partie (1) est une enveloppe (11) du dispositif orthétique ou prothétique et la seconde partie (2) est un rebord élastique (12) bordant au moins une portion de la circonférence de l'enveloppe (11) ou un segment extensible (13) inséré dans une ouverture de l'enveloppe (11).

15. Le dispositif orthétique ou prothétique avec un joint formé selon l'une quelconque des revendications précédentes, **caractérisé en ce que** la première partie (1) est l'enveloppe (11) du dispositif orthétique ou prothétique et la seconde partie (2) est une doublure flexible interne (14) avec le rebord bordant au moins une portion de la circonférence de l'enveloppe (11).
